# EUROPEAN PATENT SPECIFICATION

(11) **EP 3 437 067 B1**
(45) Date of publication and mention of the grant of the patent: **06.07.2022**
(21) Application number: 17718181.5
(22) Date of filing: 29.03.2017
(51) Int. Cl.: G06T 7/00, G01N 33/24

(54) **AUTOMATED CORE DESCRIPTION**
AUTOMATISIERTE KERNBESCHREIBUNG
DESCRIPTION DE CAROTTE AUTOMATISÉE

(30) Priority: 01.04.2016 US 201662317047 P
(43) Date of publication of application: 06.02.2019
(73) Proprietor: Saudi Arabian Oil Company, 31311 Dhahran (SA)
(72) Inventor: MEZGHANI, Mokhles Mustapha, 31311Dhahran (SA); SHAMMARI, Salem Hamoud, 31311 Dhahran (SA); ANIFOWOSE, Fatai A., 31311 Dhahran (SA)
(74) Representative: Fish & Richardson P.C.
(86) International application number: PCT/US2017/024776
(87) International publication number: WO 2017/172935

(56) References cited:
- WO-A1-2015/127349
- Angeleena Thomas ET AL: "Rock Physics and Formation Evaluation Automated lithology extraction from core photographs", First Break, 1 June 2011 (2011-06-01), XP055203531, Retrieved from the Internet: URL:http://www.geos.ed.ac.uk/homes/acurtis /Thomas_etal_FirstBreak_2011.pdf [retrieved on 2015-07-20]

## Description

### BACKGROUND

To maximize oil production and total recovery of hydrocarbons, it is important for oil companies to have a complete understanding of reservoir rocks and fluids present in producing fields. Core description is a fundamental task in reservoir characterization for predicting well properties. By analyzing collected core samples (that is, rock samples) or well logs, or both, the core description can include the description of bedding, lithology, sedimentary structures, fossils, and any other micro/macro-features of rock. Core description is usually performed by geologists and is a time-consuming task. Therefore, in practice, compared to the large number of collected core samples, only a small portion of the collected core samples are actually described by geologists.

Angeleena Thomas et al. describe in First Break, vol. 29, June 2011, on pages 103-109 an approach to lithology classification from core photographs based on object-based image analysis.

### SUMMARY

The present invention is defined by the independent claims. The dependent claims depict other embodiments of the invention. The present disclosure describes methods and systems, including computer-implemented methods, computer program products, and computer-implemented systems for automated core description.

An image associated with a core sample is received. The image represents a property of the core sample. A plurality of values of at least one image attribute are determined from the received image. A core description of the core sample is determined from a set of core descriptions (also called lithofacies). The core description describes the property of the core sample. The set of core descriptions are associated with a set of training core samples. Each training core sample has a corresponding core description and is associated with a set of plurality of values. Determining the core description of the core sample is based on a comparison between the plurality of values associated with the core sample and sets of plurality of values associated with the set of training core samples. The core description of the core sample is provided to an output device.

Some implementations can include corresponding computer-implemented systems, apparatuses, and computer programs recorded on one or more computer storage devices, each configured to perform the actions of the methods. A system of one or more computers can be configured to perform particular operations or actions by virtue of having software, firmware, hardware, or a combination of software, firmware, or hardware installed on the system that in operation causes the system to perform the actions. One or more computer programs can be configured to perform particular operations or actions by virtue of including instructions that, when executed by data processing apparatus, cause the apparatus to perform the actions.

For example, in one implementation, a computer-implemented method according to claim 1 is presented.

The foregoing and other implementations include:

Selecting the at least one image attribute based on correlations between the at least one image attribute and the property of the core sample.

Detecting, by a computer, a plurality of areas in the image, the plurality of areas corresponding to areas at a plurality of depths; determining the plurality of values of the at least one image attribute for the plurality of areas; and recording the determined plurality of values in a pseudo log, optionally wherein each of the plurality of areas represents an area in which the property of the core sample is substantially homogeneous, and the plurality of areas can be overlapping.

A second aspect, combinable with the general implementation, wherein each set of plurality of values includes values of the at least one image attribute associated with the corresponding training core sample.

A fourth aspect, combinable with the general implementation, wherein the comparison between the plurality of values associated with the core sample and sets of plurality of values associated with the set of training core samples includes determining a difference between the plurality of values and at least one set of plurality of values associated with at least one training core sample.

A fifth aspect, combinable with the general implementation, wherein determining the core description of the core sample includes providing a core description of a training core sample.

The subject matter described in this specification can be implemented in particular implementations so as to realize one or more of the following advantages. First, the described subject matter automates accurate core descriptions by applying computational intelligence (CI) techniques to high resolution or pre-processed images of core samples. As a result, subjectivity with respect to core descriptions normally generated by geologists is reduced, and core descriptions can be considered to be consistent and error-free. Second, the automation process enabled by the CI also allows a significant portion of collected core samples to be described, providing a better understanding of reservoir rocks and fluids in producing fields when compared to traditional core description methods which often sample or ignore available core sample data due to time or financial constraints, or both, of using traditional geologists. Third, the described automated core description is more efficient and faster in displaying results of depositional environments, for example, reducing a duration of a core description process from days to minutes. Fourth, the described subject matter enables geoscientists, engineers and management staffs without sedimentological skills to have a quick display of the results. Fifth, the described approach can "digitize" a geologist's experience and provide a new paradigm in a geological core description process. For example, results from the described automated process can be fed into a full-field reservoir model for reserves estimation. The results can also assist drilling engineers to plan new wells and help drillers to prepare appropriate drill bits for the estimated lithologies. When the described automated process is used real-time, it can be an integral part of a geosteering process that will assist drillers to avoid difficult formations/terrains to achieve optimum drilling experience. Other advantages will be apparent to those of ordinary skill in the art.

The details of one or more implementations of the subject matter of this specification are set forth in the subsequent accompanying drawings and the description. Other features, aspects, and advantages of the subject matter will become apparent from the description, the drawings, and the claims,

### DESCRIPTION OF DRAWINGS

FIG. 1 is a flow chart of an example method for automated core description, according to an implementation.
FIG. 2A illustrates a first core image, according to an implementation.
FIG. 2B illustrates a second core image, according to an implementation.
FIG. 3A illustrates a first Formation Micro Imager (FMI) image, according to an implementation.
FIG. 3B illustrates a second FMI image, according to an implementation.
FIG. 3C illustrates a third FMI image, according to an implementation.
FIG. 4 illustrates an example pseudo log, according to an implementation.
FIG. 5 is a block diagram illustrating an exemplary distributed computer-implemented system (EDCS) used to provide automated core description, according to an implementation.

Like reference numbers and designations in the various drawings indicate like elements.

### DETAILED DESCRIPTION

The present detailed description relates to automated core description and is presented to enable any person skilled in the art to make, use, or practice the disclosed subject matter, and is provided in the context of one or more particular implementations. Various modifications to the disclosed implementations will be readily apparent to those skilled in the art, and the general principles defined herein may be applied to other implementations and applications without departing from scope of the disclosure. Thus, the present disclosure is not intended to be limited to the described or illustrated implementations, but is to be accorded the widest scope consistent with the principles and features disclosed herein as defined by the appended claims.

A core sample is a piece of rock (for example, cylindrical in shape and of varying lengths) including one or more lithofacies extracted from a wellbore beneath the earth's surface that provides actual/accurate physical evidence of reservoir formation characteristics, for example, rock type, formation thickness, grain size, or permeability (that is, ability of the rock to permit fluid flow). In some instances, core samples can also reveal structural dip, fault, fracture, porosity, mineral composition, or other values or conditions. Core description is a fundamental task in reservoir characterization for predicting well properties and typically includes descriptions of bedding, lithology, sedimentary structures, fossils, and any other micro/macro-features of rocks. For example, lithology can include characteristics such as color, texture, and grain size of rock. Typically, core description is performed by a geologist who observes a physical core sample, high resolution image of a core sample, or analyzes well logs that were obtained during wellbore drilling or after the drilling is complete.

Two types of images are typically associated with a core sample: a core image and a borehole image. A core image is an image (for example, in a digital graphics format such as JPG/JPEG, GIF, BMP, TIFF, PNG, AVI, DV, MPEG, MOV, WMV, or RAW) of a particular core sample taken by a high-resolution camera (such as a digital camera). After the core sample has been collected, the core sample can be taken to a lab, cleaned, and an image taken to preserve data associated with the core sample in case the core sample is damaged or moved to a remote storage location. Since the core sample is cleaned and the image is taken in a lab, the core image is typically in high-resolution. In typical implementations, the core image can be taken from one or more angles or as a 360-degree (full circumference) image. For example, a 360-degree image can be obtained by rotating the core sample in relation to a fixed camera or moving the camera around the stationary core sample. The 360-degree image can then be processed and treated as a two-dimensional (for example, rectangular) image of a three-dimensional object (the core sample). In another example, images can be taken of a flat surface of a slabbed core sample under controlled conditions of light and orientation.

On the other hand, a borehole image shows the rock left around the perimeter of the wellbore and can be obtained either in real-time during the wellbore drilling or after drilling is complete. Borehole images can be acquired through different types of logging tools transmitting and receiving signals (for example, acoustic, radio, or signals) into and from, respectively, the wellbore. The borehole image can also be considered as a well log (that is, an image log). One example of a borehole image is a Formation Micro Imager (FMI) image. Typically, the resolution of the borehole image is not as good as that of the core image, because image quality can be adversely impacted due to camera instability, dirt, seeping materials (for example, water, oil, or gas) from the wellbore walls, or other factors. This is especially true if captured in real-time during or immediately following drilling operations.

The described automated core description system can automatically predict core sample properties based on information associated with the core sample. Some existing approaches for automated core description use conventional well logs which do not include image logs such as borehole images in combination with computational intelligence (CI) techniques. For example, commercial software packages such as GEOLOG and TECHLOG have been designed to provide this application. However, core images or borehole images have not been used in combination with CI to provide automated core description because the core images or borehole images are not in a native input format to be used by CI. The CI tools normally take an input format of numerical values representing an attribute used to predict core sample properties. While the image is a generated representation/visualization of the core sample or the borehole, the image itself does not provide numerical values of an attribute that can be used by CI tools.

The described approach provides automated core description by combining CI with image processing. Core images or borehole images can be converted to numerical values of attributes using techniques of image processing, statistical analysis, pattern recognition, or others. The converted numerical values of each attribute can be collected together and treated as a conventional well log and served as inputs to the CI.

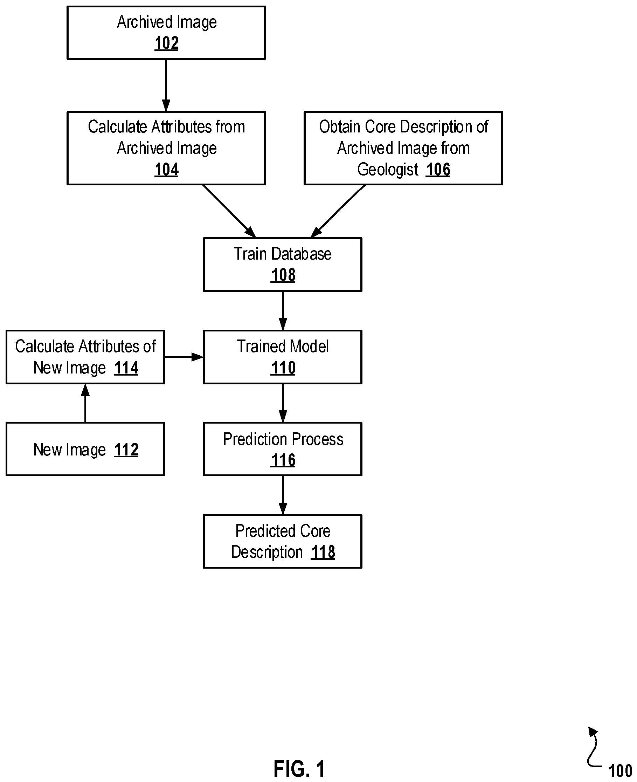

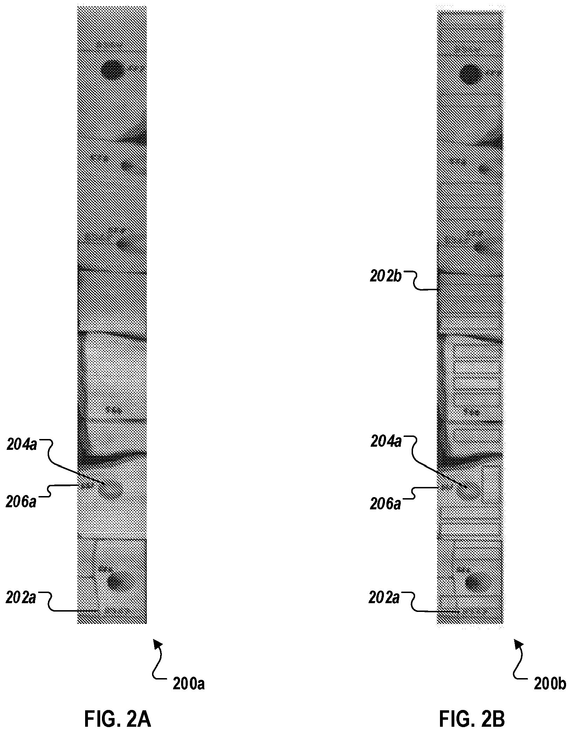

FIG. 1 is a flow chart of an example method 100 for automated core description, according to an implementation. The method 100 can be performed by an automated core description system as described in this disclosure. For clarity of presentation, the description that follows generally describes method 100 in the context of FIGS. 2A-2B, 3A-3C, 4, and 5. However, it will be understood that method 100 may be performed, for example, by any suitable system, environment, software, and hardware, or a combination of systems, environments, software, and hardware as appropriate. In some implementations, various steps of method 100 can be run in parallel, in combination, in loops, or in any order.

At 102, an archived image, whose core sample has already has been described by a geologist is received. In some cases, the core sample of the archived image may have not been described by a geologist. In typical implementations, the archived image can be a core image or a borehole image as described. In other implementations, the archived image can be any image providing data consistent with this disclosure (for example, a video image or data associated with an archived image).

For example, turning to FIG. 2A, FIG. 2A illustrates a first core image 200a, according to an implementation. The core image 200a represents a contiguous core sample of rock (for example, of about 12 feet long that can be used for image analysis to determine one or more attributes. The illustrated first core image 200a includes depth data 202a (here an alphanumeric label), plug area 204a (that is, areas that plug samples are taken from the rock in the lab for enhanced analysis), and a plug identity 206a (here an alphanumeric label).

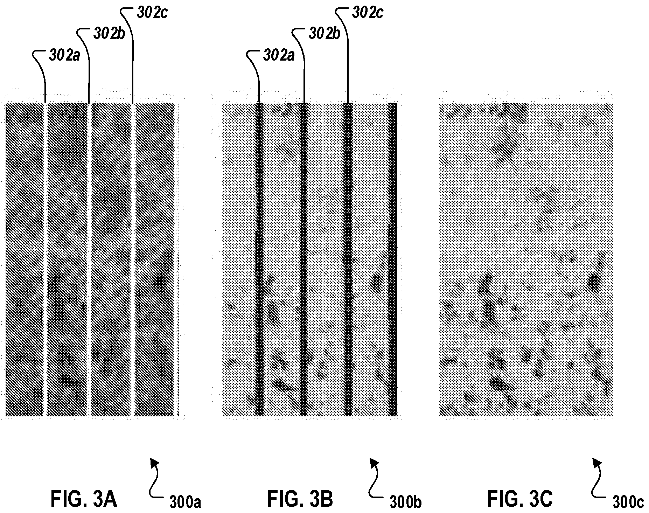

Turning to FIG. 3A, FIG. 3A illustrates a first FMI image 300a, according to an implementation. The FMI image 300a can also be used for image data and, as described previously, represents the rock left around the perimeter of the wellbore. As illustrated, the FMI image 300a includes stripes 302a-302c that are typical in an FMI image configuration and represent missing data. For example, each stripe 302a-302c can represent a particular pad or an arm of a tool that is used to acquire the FMI image. Returning to FIG. 1, from 104, method 100 proceeds to 106

At 104, attributes from archived images (for example, FIGS. 2A and 3A) can be computed. Multiple images from different depths of the same wellbore, can be stacked together. As illustrated in FIG. 2A, the illustrated core image 200a can be marked with corresponding depth values (for example, depth data 202a). These core images can be sequentially arranged or stacked together in the order of depth to form a contiguous profile corresponding to the cored sections of a reservoir. In other words, multiple core images, from the same wellbore, can be arranged vertically end-to-end by depth to form a contiguous profile of a well. In some implementations, an optical character recognition (OCR) algorithm can be used to obtain and process data such as the depth data 202a, plug identity 206a, or other such data consistent with this disclosure. While the FMI wellbore images of FIG. 3A do not have illustrated depth values, the same stacking to form continuous profiles can also be performed using contiguous FMI wellbore images.

In some implementations, the core images can be named with start depth and end depth information. The depth information can be retrieved automatically during image loading (regardless of image order loading) and used to depth-match extracted attributes from a core image. The extracted depth information can be used to sort extracted attributes to form a continuous profile of a wellbore as pseudo logs.

To facilitate attribute computation, core images can be pre-processed. For example, multi-point statistics (MPS) techniques can be used to fill the missing data of the core images. MPS can be used to fill plug areas (for example, plug area 204a in FIG. 2A) as well as some missing parts (for example, cracks and broken parts of the core sample as illustrated in FIGS. 2A and 2B). MPS is a technique that estimates the conditional probabilities at desired points given the observed data at neighboring points. In some implementations, during the pre-processing, the core image can be normalized, as will be discussed subsequently, so that pixel intensities in the image are normalized to a certain range. Other image pre-processing techniques, such as noise reduction techniques or other pre-processing, can also be used.

To help computing attributes, areas used for attribute computation can be identified. The identified areas may exclude areas with non-geologically related features (for example, plug area or painted tags). In some implementations, if, for example, cracks and plug areas in a core image have been filled in by pre-processing techniques such as MPS, these filled-in areas can also be used for attribute computation. In some implementations, if the automated core description is to be used to describe core lithology, during the pre-processing step the core image can be reconstructed to avoid processing areas for data not reflecting core lithology, and the reconstructed core image may then be used for attribute computation. Additionally, the pre-processed core image can be quality-controlled to ensure that no non-geological or incorrect features have been introduced by pre-processing operations.

Turning to FIG. 2B, FIG. 2B illustrates a second core image 200b, according to an implementation. The core image 200b represents the same core sample as that in FIG. 2A with areas to be used for attribute computation identified by windows 202b. As illustrated, the identified areas can exclude various areas (here, plug areas 204a and painted tags such as depth data 202a and plug identities 206a). The windows 202b can specify regions to be covered by the core description. In some instances, after pre-processing (as previously described), the entire core image or reconstructed core image can be used for attribute computation. Each window 202b can also include a region with homogeneous (similar) core property. Multiple windows 202b can be defined along an image from the beginning of the core image to the end. The multiple windows 202b can be non-overlapping or overlapping. In some implementations, each window 202b can have a fixed window size or a variable window size (for example, user defined or determined automatically by a particular area of the core image or attribute-of-interest type). In some typical implementations, the window 202b can be a rectangular window that spans the entire width of the core image or as much of the width of the core image as possible. The window 202b may have a shape other than rectangular. In some implementations, the window 202b size (for example, height or width, or both) can be an input parameter (automated or manual entry) to the automated core description system and can be changed during the described process. In some implementations, the automated core description system can automatically detect or suggest, or both, an optimal window 202b size (for example, where the core properties are homogeneous within the window 202b.)

In some implementations, a moving window 202b can be used. The moving window 202b can be visualized as a fixed-size window 202b moving with a small step (for example, manually or dynamically determined) along the core sample 200b to form a series of overlapped windows 202b. For example, for a one-inch high moving window 202b and a moving step of 0.2 inches, the series of overlapped windows can include a first window 202b covering the core sample area from depth X inch to X+1 inch, a second window from depth X+0.2 inch to X+1.2 inch, a third window from depth X+0.4 inch to X+1.4 inch, and so on.

Returning to FIG. 1, at 104, as previously described, well logs can also be used by the described automated core description system, and can include borehole images such as FMI images and conventional well logs such as logs of density, oil saturation, neutron, water saturation, gamma ray, sonic, resistivity, and other types of data. A number of linear and non-linear feature selection techniques can be applied to select logs that are correlated with the core properties to be predicted. These techniques could be neural network, decision tree, Markov chain, or others. The selected logs will be used by the described automated core description system. For example, if the automated core description system is designed to predict lithology, logs with physical measurements that depend on lithology can be selected. This can include FMI images with attributes that relate to texture such as homogeneity, orientation, and entropy and conventional logs of gamma ray, sonic, neutron, and density data.

Well logs can be pre-processed, for example, to normalize the logs, to discard low quality measurements, and to fill the missing data for an FMI image using multi-points statics (MPS) techniques (such as the image of FIG. 3A). Normalization is used if the conventional log contains one or more attributes that have large variations (for example, in the order of thousands), the log data can be normalized so that the data is within a certain range. The FMI image can also be normalized if the image values have a large variation. Examples of normalization can include log function which transforms numbers to the natural log scale or a normalization function which may scale the numbers to a range from -1 to +1. A threshold can be used to determine if measurements are of low quality and to decide whether to discard.

Turning to FIG. 3B, FIG. 3B illustrates a second FMI image 300b, according to an implementation. The FMI image 300b can represent a normalized FMI image (including stripes 302a-302c as in FIG. 3A) with the image values (for example, image pixel intensity values) within a certain range. The FMI image 300b may represent an image enhanced by an image processing feature and coloration to distinguish different rock characteristics.

Turning to FIG. 3C, FIG. 3C illustrates a third FMI image 300c, according to an implementation. The FMI image 300c can represent an FMI image in which the missing data (represented by stripes 302a-302c in FIGS. 3A and 3B) has been filled in by a pre-processing technique (for example, MPS). Similar to a core image as in FIG. 2A, areas in an FMI image used for attribute computation can be identified. As stated previously, multiple windows (similar to illustrated rectangular window 202b but not illustrated in FIG. 3C) can be defined in the FMI image 300c to facilitate attribute computation.

Returning to FIG. 1, at 104, the automated core description system can choose a set of image attributes to be computed. For example, the attributes can be chosen based on correlations to the core properties to be predicted. Attributes that relate to the geological features of the images can be chosen such as color, texture, orientation, and size and distribution of the grains of the images. In some cases, to achieve a good core prediction performance, conventional logs such as density, sonic and gamma ray may also be included. A number of linear or non-linear feature selection techniques can be used to select the set of attributes. These techniques could be neural network, decision tree, Markov chain, or others. Table 1 lists examples of attributes, with definitions, that can be computed from the core image. These attributes pertain to core image color, intensity, texture, distribution and orientation. As will be appreciated by those of ordinary skill in the art, other attributes consistent with this disclosure can also be used. The example list of attributes in Table 1 is not meant to limit the described subject matter in any way.

**Table 1: Attributes Computed from Core Images**

| | **Image Attributes** | **Definition** |
|---|---|---|
| 1. | MaxIntensity | The value of the pixel with the greatest intensity in the region. |
| 2. | MeanIntensity | The mean of all the intensity values in the region. |
| 3. | MinIntensity | The value of the pixel with the lowest intensity in the region. |
| 4. | PowerIntensity | The value indicating the intensity power of image. |
| 5. | MedianIntensity | The median of all the intensity values in the region. |
| 6. | StandardDeviationIntensity | The standard deviation of all the intensity values in the region. |
| 7. | Entropy | Statistical measure of randomness that can be used to characterize the texture of the input image. |
| 8. | Area | Number of grains present in the image. |
| 9. | Perimeter | Average size of objects present in binary image. |
| 10. | Contrast | A measure of the intensity contrast between a pixel and its neighbor over the whole image. |
| 11. | Correlation | A measure of how correlated a pixel is to its neighbor over the whole image. |
| 12. | Homogeneity | A measure of the closeness of the distribution of grains. |
| 13. | Orientation | A measure of the degree of lamination showing the orientation of the grains between the horizontal and the vertical direction (measured in degrees, ranging from -90 to 90 degrees). |

In typical implementations, various image processing techniques (for example, commercial or proprietary, or both) can be used to compute numerical values of attributes for the identified areas in the core image or borehole image. In some implementations, attributes can be computed based on analyzing the image pixel intensities within a window (for example, 202b). For each attribute, an attribute value is computed for a particular window 202b. In case of a moving window 202b, an attribute value is computed for each overlapping window 202b. The attribute value can be associated with the depth information of the window 202b. Since each window 202b can cover a core sample area of a certain range in depth, the depth of the window 202b can be chosen as the smallest depth value, the largest depth value, the average depth value of the covered core sample area, or other depth values that are apparent to those of ordinary skill in the art. The attribute values of windows 202b along the core sample can be collected to form a pseudo log. In other words, the pseudo log can include attribute values at different depths. The attribute values are in numerical format. The pseudo log can be stored in a spreadsheet, a table, a matrix, or other formats.

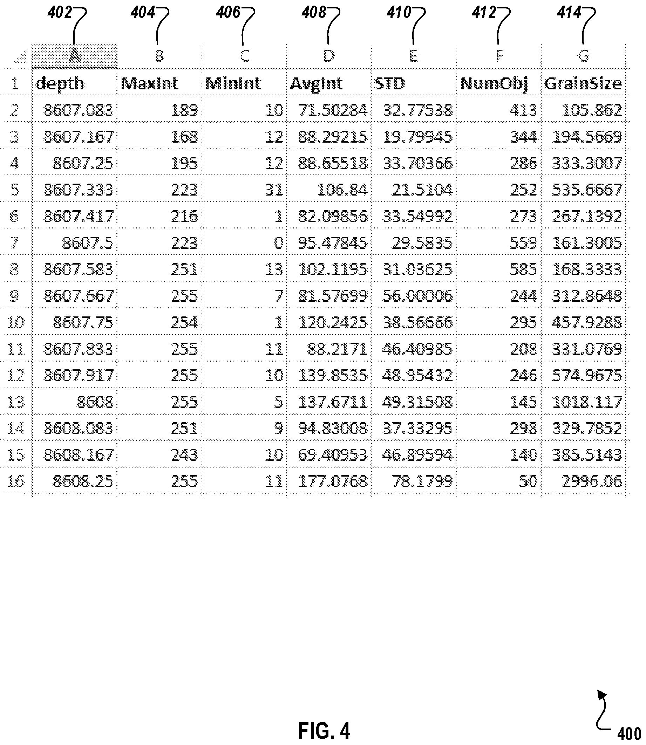

Turning to FIG. 4, FIG. 4 illustrates an example pseudo log 400, according to an implementation. The pseudo log 400 captures image attribute values at different depths. Each row of pseudo log 400 represents one depth. The pseudo log 400 contains a depth column 402 and six columns of image attributes, including maximum pixel intensity 404, minimum pixel intensity 406, average pixel intensity 408, number of objects 410, and grain size 412. As will be appreciated by those of ordinary skill in the art, formats other than those shown in pseudo log 400 can also be used. In some implementations, one pseudo log can be generated for each attribute. The pseudo log can then be treated as a conventional well log to work with the previously described CI. In some implementations, a number of linear and non-linear feature selection techniques can be applied to select a set of pseudo logs that are correlated with the core properties to be predicted. These techniques could be neural network, decision tree, Markov chain, or others. The selected pseudo logs can then be used by the automated core description system to predict core properties. Returning to FIG. 1, from 104, method 100 proceeds to 108.

At 106, a geologist's core description of the core sample associated with the received archived image at 102 can also be obtained. For the purposes of method 100, the geologist's core description is considered to be an accurate core description. Depending on a particular implementation, the geologist's core description can be given less, equal, or more weight than calculated attributes from an archived image. From 106, method 100 also proceeds to 108.

At 108, a memory (for example, a conventional, in-memory, or other database) used by CI techniques to automate core descriptions is created and used to train the CI techniques. The memory can store the pseudo logs of image attributes obtained at 104, the geologist's core description obtained at 106, and other data consistent with this disclosure (for example, executable stored procedures, particular wellbore data, or particular field data). In some implementations, the memory can also store the conventional well logs correlated to the core properties to be predicted. For example, 102, 104, and 106 of method 100 can be performed for each archived image whose core sample has been described by the geologist. The accurately described core samples can serve as training samples and stored in the memory. The core sample that can be served as a training sample is also called training core sample. The CI techniques can learn patterns from the training samples (that is, in the memory is trained) and predict core properties of a new core sample. Each training sample contains information of one accurately described core sample and includes a pair of input and output, where the input can include the pseudo logs of image attributes or the conventional well logs, or both, and the output can include the accurate core description from the geologist. For example, the automated core description system can be designed to predict grain size of the rock and classify the grain size to three classes: large, medium, and small. The memory can include training samples with grain sizes that have already been accurately classified by the geologist. The input of each training sample can include the pseudo logs of image attributes or conventional well logs correlated to grain size, or both, and the output is then the accurate grain size from the geologist. As will be discussed subsequently, when the automated core description system receives a new core sample which has not been described by the geologist, the CI can compare the new core sample with the training samples in the memory and automatically classify the grain size of the new core sample.

Various CI techniques can be used to train the memory and automate core description, for example, data mining, artificial intelligence, pattern recognition, machine learning, neutral networks, decision trees, support vector machines, hybrid and ensemble machine learning, or techniques that are apparent to those of ordinary skill in the art and consistent with this disclosure. For example, a neural network can include supervised, unsupervised, or reinforcement approaches. In the presence of a large volume of core samples already described by geologists, a supervised approach can be used because the CI can learn patterns well from the large volume of the accurately described core samples and predict the core description of a new core sample. In some instances, a clustering algorithm can be used to suggest possible core description for the new core sample.

In some implementations, core samples of the same rock formation can be in one memory while core samples of a different rock formation can form a separate memory. Normally core samples from the same well or core samples from different wells but with the same rock formation are in one memory. If a new well is drilled in a new area, a new memory may be created if the rock formation of the new area is different (or varies beyond a defined threshold value) from the rock formations of the existing memories. From 108, method 100 proceeds to 110.

At 110, a trained model is obtained based on CI and the training samples. The trained model can be used (for example, as previously described) to automatically describe new core samples that have not been described by the geologist. From 110, method 100 proceeds to 116.

At 112, a new image, whose core sample has not been described by the geologist, is obtained (using a new image/attributes as described in 112 and 114 subsequently). In typical implementations, the image is a core image or a borehole image. From 108, method 100 proceeds to 114.

At 114, image attributes can be computed from the new image obtained at 112. In typical implementations, a similar approach to that described with respect to 104 is used. For example, the image can be pre-processed to fill up missing data, areas for attribute computation can be identified, image attributes at different depths can be computed for the identified areas, and pseudo logs of image attributes can be generated. The generated pseudo logs can be sent to the trained model at 110 for automated core description. Those of ordinary skill in the art will realize that subtle variations from 104 in the processing of the new image obtained at 112 could occur. From 114, method 100 proceed to 110.

At 116, CI techniques (for example, similar to those of 108) can be used to predict core description of a new core sample. The new core sample can be compared with the training samples in the memory, and a training sample "closest" (for example, based on some determined range or scale) to the new core sample can be identified. The core description of the closest training sample can then be used to serve as the core description of the new core sample. In determining the closest training sample, the difference (for example, Euclidean distance) between the pseudo logs of the new core sample and the pseudo logs of each training sample can be computed. In some cases, the difference between the conventional logs of the new core sample and the conventional logs of each training sample can be computed. The closest training sample can be the one with the minimum difference to the new core sample. In some instances, ambiguity can arise if the new core sample has similar differences to multiple training samples of different core descriptions. In this case, the new core sample can be passed to the geologist for an accurate core description or an algorithm can be used to disambiguate or choose accurate value based on other available data consistent with this disclosure (for example, location in a well field, or other known core descriptions of nearby wells/similar depth). The accurately described core sample can then be included in the memory as a training sample. From 116, method 100 proceed to 118.

At 118, the predicted core description of the new core sample is obtained. At the testing stage of the automated core description system, the predicted core description can be passed to a geologist for validation. In some implementations, the validated core description can be further included as a training example. The described methodology helps to improve the consistency, accuracy, objectivity, repeatability, speed, and efficiency of the core description process.

At the operational stage, the predicted core description can be used by geologists, petroleum engineers, and exploration teams for further reservoir characterization processes to predict, among other things, reservoir flow properties, volumes, and fluid saturations. For example, predicted core descriptions can provide accurate information about lithofacies. Accurate lithofacies information provides porosity and permeability values, two of the most important parameters required for volumetric estimation of a reservoir. When a predicted core description is imported into other applications such as PETREL, GEOLOG, a basin model, or a reservoir simulator, a generated predictive sedimentary environment can be used as a parameter required to populate reservoir models and to predict reservoir discoveries.

The predicted core description can also be used to maximize chances of drilling success to mitigate loss of production resources. For example, predictive data can be used to influence (including in real-time) drilling operations (for example, drill direction, depth, speed, and methods), drilling locations, pumping operations (for example, volume and rate), and refining operations.

In some implementations, the automated core description system can be implemented in computing languages such as C, C++, or JAVA, or a proprietary commercial programming language such as MATLAB. The use of these, or other computing languages and software tools can assist subject matter experts in decision making and generating modeling tools.

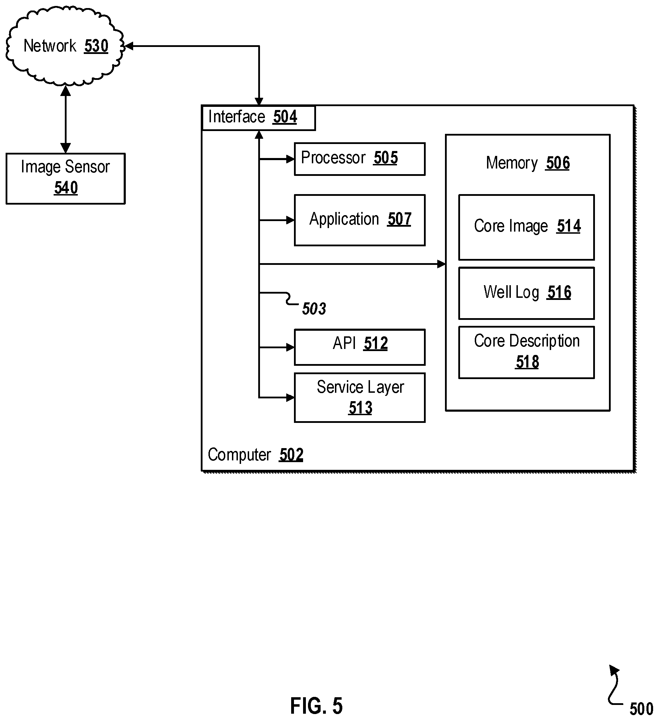

Turning to FIG. 5, FIG. 5 is a block diagram illustrating an exemplary distributed computer-implemented system (EDCS) 500 used to provide automated core description according to an implementation. In some implementations, the EDCS 500 includes a computer 502, network 530, and image sensor 540.

The illustrated computer 502 is intended to encompass a computing device such as a server, desktop computer, laptop/notebook computer, wireless data port, smart phone, personal data assistant (PDA), tablet computing device, one or more processors within these devices, or any other suitable processing device, including physical or virtual, or both, instances of the computing device. The computer 502 may comprise a computer that includes an input device, such as a keypad, keyboard, touch screen, or other device (not illustrated) that can accept user information, and an output device (not illustrated) that conveys information associated with the operation of the computer 502, including digital data, visual or audio, or both, information, or a user interface.

The computer 502 can serve as a client or a server, or both. In typical implementations, the computer 502 act as either a parallel processing node, host for a software agent, host for a database, CI application(s), image processor(s), user interface, automated core description application, or other application consistent with this disclosure (even if not illustrated). The illustrated computer 502 is communicably coupled with a network 530. In some implementations, one or more components of the computer 502 may be configured to operate within a parallel-processing or cloud-computing-based, or both, environment. Implementations of the computer 502 can also communicate using message passing interface (MPI) or other interface over network 530.

At a high level, the computer 502 is an electronic computing device operable to receive, transmit, process, store, or manage data and information associated with automated core description. According to some implementations, the computer 502 may also include or be communicably coupled with a simulation server, application server, e-mail server, web server, caching server, streaming data server, or other server.

The computer 502 can receive requests over the network 530 from an application 507 (for example, executing on another computer 502) and respond to the received requests by processing the said requests in an appropriate software application 507. In addition, requests may also be sent to the computer 502 from internal users (for example, from a command console or by other appropriate access method), external or third-parties, other automated applications, as well as any other appropriate entities, individuals, systems, or computers.

Each of the components of the computer 502 can communicate using a system bus 503. In some implementations, any or all the components of the computer 502, hardware or software, or both, may interface with each other or the interface 504 over the system bus 503 using an application programming interface (API) 512 or a service layer 513, or both. The API 512 may include specifications for routines, data structures, and object classes. The API 512 may be either computer-language independent or dependent and refer to a complete interface, a single function, or even a set of APIs. The service layer 513 provides software services to the computer 502 or system of which the computer 502 is a part. The functionality of the computer 502 may be accessible for all service consumers using this service layer. Software services, such as those provided by the service layer 513, provide reusable, defined functionalities through a defined interface. For example, the interface may be software written in JAVA, C++, or suitable languages providing data in extensible markup language (XML) format or other suitable formats. While illustrated as an integrated component of the computer 502, alternative implementations may illustrate the API 512 or the service layer 513, or both, as stand-alone components in relation to other components of the computer 502. Moreover, any or all parts of the API 512 or the service layer 513, or both, may be implemented as child or sub-modules of another software module, enterprise application, or hardware module without departing from the scope of this disclosure.

The computer 502 includes an interface 504. Although illustrated as a single interface 504 in FIG. 5, two or more interfaces 504 may be used according to particular needs, desires, or particular implementations of the computer 502. The interface 504 is used by the computer 502 for communicating with other systems in a distributed environment - including a parallel processing environment - connected to the network 530 (whether illustrated or not). Generally, the interface 504 comprises logic encoded in software or hardware, or both, in a suitable combination and operable to communicate with the network 530. More specifically, the interface 504 may comprise software supporting one or more communication protocols associated with communications over network 530.

The computer 502 includes a processor 505. Although illustrated as a single processor 505 in FIG. 5, two or more processors may be used according to particular needs, desires, or particular implementations of the computer 502. Generally, the processor 505 executes instructions and manipulates data to perform the operations of the computer 502. Specifically, the processor 505 executes the functionality required for automated core description.

The computer 502 also includes a memory 506 that holds data for the computer 502 or other components of a system of which the computer is a part. The memory 506 can be considered to be a database, system memory, other forms of memory, or a combination of one or more of these. For example, the memory 506 can be a database that holds data required for automated core description. Although illustrated as a single memory 506 in FIG. 5, two or more memories may be used according to particular needs, desires, or particular implementations of the computer 502. While memory 506 is illustrated as an integral component of the computer 502, in alternative implementations, memory 506 can be external to the computer 502. In some implementations, memory 506 can hold or reference one or more of a core image 514, well log 516, or core description 518.

The application 507 is an algorithmic software engine providing functionality according to particular needs, desires, or particular implementations of the computer 502 or a system of which the computer 502 is a part, particularly with respect to functionality required for automated core description. For example, application 507 can serve as (or a portion of) a database, CI application(s), image processor(s), user interface, automated core description application, or application consistent with this disclosure (whether illustrated or not). Although illustrated as a single application 507, the application 507 may be implemented as multiple applications 507 on the computer 502. In addition, although illustrated as integral to the computer 502, in alternative implementations, the application 507 can be external to and execute apart from the computer 502.

There may be any number of computers 502 associated with a computer-implemented system performing functions consistent with this disclosure. Further, the term "client," "user," and other appropriate terminology may be used interchangeably as appropriate without departing from the scope of this disclosure. Moreover, this disclosure contemplates that many users/processes may use one computer 502, or that one user/process may use multiple computers 502.

Image sensor 540 is operable to at least capture an image of a core sample. In some implementations, image sensor 540 can use a lens assembly to focus light onto an electronic image sensor and digitally record image information into a memory (not illustrated) in various digital file formats. Examples of digital file formats used to record the image information can include JPG/JPEG, GIF, BMP, TIFF, PNG, AVI, DV, MPEG, MOV, WMV, or RAW. In some implementations, the electronic image sensor can be a charge coupled device (CCD), an active pixel sensor (CMOS), or other suitable electronic image sensor. Image sensor 540 may provide a live preview of the external image source to be photographed. Image sensor 540 may also provide optical or digital, or both, zoom functionality and panoramic images in both two and three dimensions. In other implementations, the recorded image information can be both still and video with sound. In some implementations, image sensor 540 can be a non-digital image sensor that can take images that can subsequently be scanned or processed in to digital images for use by the described subject matter.

In some implementations, image data recorded by image sensor 540 may also be transferred over network 530 to a remote data storage location (not illustrated) instead of being stored in memory 506. Although illustrated as communicably connected (for example, by a cable or wireless connection) through network 530 to computer 502, in some implementations, image sensor 540 may also be integrated into computer 502 or other components (not illustrated) of computer-implemented system 500 or directly connected to an interface port (not illustrated) on computer 502. While the computer-implemented system 500 is illustrated as containing a single image sensor 540, alternative implementations of computer-implemented system 500 may include any number of image sensors 540, working individually or in concert, and suitable to the purposes of the EDCS 500. In some implementations, image sensor(s) 540 can be part of a mechanical assembly (not illustrated) for moving, adjusting, or stabilizing the image sensor(s) 540 or a core sample to obtain the image of the core sample.

Implementations of the subject matter and the functional operations described in this specification can be implemented in digital electronic circuitry, in tangibly embodied computer software or firmware, in computer hardware, including the structures disclosed in this specification and their structural equivalents, or in combinations of one or more of them. Implementations of the subject matter described in this specification can be implemented as one or more computer programs, that is, one or more modules of computer program instructions encoded on a tangible, non-transitory, computer-readable computer-storage medium for execution by, or to control the operation of, data processing apparatus. Alternatively, or additionally, the program instructions can be encoded in/on an artificially generated propagated signal, for example, a machine-generated electrical, optical, or electromagnetic signal that is generated to encode information for transmission to suitable receiver apparatus for execution by a data processing apparatus. The computer-storage medium can be a machine-readable storage device, a machine-readable storage substrate, a random or serial access memory device, or a combination of computer-storage mediums.

The term "real-time," "real time," "realtime," "real (fast) time (RFT)," "near(ly) real-time (NRT)," "quasi real-time," or similar terms (as understood by one of ordinary skill in the art), means that an action and a response are temporally proximate such that an individual perceives the action and the response occurring substantially simultaneously. For example, the time difference for a response to display (or for an initiation of a display) of data following the individual's action to access the data may be less than 1 ms, less than 1 sec., less than 5 secs., etc. While the requested data need not be displayed (or initiated for display) instantaneously, it is displayed (or initiated for display) without any intentional delay, taking into account processing limitations of a described computing system and time required to, for example, gather, accurately measure, analyze, process, store, or transmit the data.

The terms "data processing apparatus," "computer," or "electronic computer device" (or equivalent as understood by one of ordinary skill in the art) refer to data processing hardware and encompass all kinds of apparatus, devices, and machines for processing data, including by way of example, a programmable processor, a computer, or multiple processors or computers. The apparatus can also be or further include special purpose logic circuitry, for example, a central processing unit (CPU), an FPGA (field programmable gate array), or an ASIC (application-specific integrated circuit). In some implementations, the data processing apparatus or special purpose logic circuitry (or a combination of the data processing apparatus or special purpose logic circuitry) may be hardware- or software-based (or a combination of both hardware- and software-based). The apparatus can optionally include code that creates an execution environment for computer programs, for example, code that constitutes processor firmware, a protocol stack, a database management system, an operating system, or a combination of execution environments. The present disclosure contemplates the use of data processing apparatuses with or without conventional operating systems, for example LINUX, UNIX, WINDOWS, MAC OS, ANDROID, IOS, or any other suitable conventional operating system.

A computer program, which may also be referred to or described as a program, software, a software application, a module, a software module, a script, or code can be written in any form of programming language, including compiled or interpreted languages, or declarative or procedural languages, and it can be deployed in any form, including as a stand-alone program or as a module, component, subroutine, or other unit suitable for use in a computing environment. A computer program may, but need not, correspond to a file in a file system. A program can be stored in a portion of a file that holds other programs or data, for example, one or more scripts stored in a markup language document, in a single file dedicated to the program in question, or in multiple coordinated files, for example, files that store one or more modules, sub-programs, or portions of code. A computer program can be deployed to be executed on one computer or on multiple computers that are located at one site or distributed across multiple sites and interconnected by a communication network. While portions of the programs illustrated in the various figures are shown as individual modules that implement the various features and functionality through various objects, methods, or other processes, the programs may instead include a number of sub-modules, third-party services, components, libraries, and such, as appropriate. Conversely, the features and functionality of various components can be combined into single components as appropriate. Thresholds used to make computational determinations can be statically, dynamically, or both statically and dynamically determined.

The methods, processes, logic flows, etc. described in this specification can be performed by one or more programmable computers executing one or more computer programs to perform functions by operating on input data and generating output. The methods, processes, logic flows, etc. can also be performed by, and apparatus can also be implemented as, special purpose logic circuitry, for example, a CPU, an FPGA, or an ASIC.

Computers suitable for the execution of a computer program can be based on general or special purpose microprocessors, both, or any other kind of CPU. Generally, a CPU will receive instructions and data from a read-only memory (ROM) or a random access memory (RAM), or both. The essential elements of a computer are a CPU, for performing or executing instructions, and one or more memory devices for storing instructions and data. Generally, a computer will also include, or be operatively coupled to, receive data from or transfer data to, or both, one or more mass storage devices for storing data, for example, magnetic, magneto-optical disks, or optical disks. However, a computer need not have such devices. Moreover, a computer can be embedded in another device, for example, a mobile telephone, a personal digital assistant (PDA), a mobile audio or video player, a game console, a global positioning system (GPS) receiver, or a portable storage device, for example, a universal serial bus (USB) flash drive, to name just a few.

Computer-readable media (transitory or non-transitory, as appropriate) suitable for storing computer program instructions and data include all forms of non-volatile memory, media and memory devices, including by way of example semiconductor memory devices, for example, erasable programmable read-only memory (EPROM), electrically erasable programmable read-only memory (EEPROM), and flash memory devices; magnetic disks, for example, internal hard disks or removable disks; magneto-optical disks; and CD-ROM, DVD+/-R, DVD-RAM, and DVD-ROM disks. The memory may store various objects or data, including caches, classes, frameworks, applications, backup data, jobs, web pages, web page templates, database tables, repositories storing dynamic information, and any other appropriate information including any parameters, variables, algorithms, instructions, rules, constraints, or references thereto. Additionally, the memory may include any other appropriate data, such as logs, policies, security or access data, reporting files, as well as others. The processor and the memory can be supplemented by, or incorporated in, special purpose logic circuitry.

To provide for interaction with a user, implementations of the subject matter described in this specification can be implemented on a computer having a display device, for example, a CRT (cathode ray tube), LCD (liquid crystal display), LED (Light Emitting Diode), or plasma monitor, for displaying information to the user and a keyboard and a pointing device, for example, a mouse, trackball, or trackpad by which the user can provide input to the computer. Input may also be provided to the computer using a touchscreen, such as a tablet computer surface with pressure sensitivity, a multi-touch screen using capacitive or electric sensing, or other type of touchscreen. Other kinds of devices can be used to provide for interaction with a user as well; for example, feedback provided to the user can be any form of sensory feedback, for example, visual feedback, auditory feedback, or tactile feedback; and input from the user can be received in any form, including acoustic, speech, or tactile input. In addition, a computer can interact with a user by sending documents to and receiving documents from a device that is used by the user; for example, by sending web pages to a web browser on a user's client device in response to requests received from the web browser.

The term "graphical user interface," or "GUI," may be used in the singular or the plural to describe one or more graphical user interfaces and each of the displays of a particular graphical user interface. Therefore, a GUI may represent any graphical user interface, including but not limited to, a web browser, a touch screen, or a command line interface (CLI) that processes information and efficiently presents the information results to the user. In general, a GUI may include a plurality of user interface (UI) elements, some or all associated with a web browser, such as interactive fields, pull-down lists, and buttons. These and other UI elements may be related to or represent the functions of the web browser.

Implementations of the subject matter described in this specification can be implemented in a computing system that includes a back-end component, for example, as a data server, or that includes a middleware component, for example, an application server, or that includes a front-end component, for example, a client computer having a graphical user interface or a Web browser through which a user can interact with an implementation of the subject matter described in this specification, or any combination of one or more such back-end, middleware, or front-end components. The components of the system can be interconnected by any form or medium of wireline or wireless digital data communication (or a combination of data communication), for example, a communication network. Examples of communication networks include a local area network (LAN), a radio access network (RAN), a metropolitan area network (MAN), a wide area network (WAN), Worldwide Interoperability for Microwave Access (WIMAX), a wireless local area network (WLAN) using, for example, 802.11 a/b/g/n or 802.20 (or a combination of 802.11x and 802.20 or other protocols consistent with this disclosure), all or a portion of the Internet, or any other communication system or systems at one or more locations (or a combination of communication networks). The network may communicate with, for example, Internet Protocol (IP) packets, Frame Relay frames, Asynchronous Transfer Mode (ATM) cells, voice, video, data, or other suitable information (or a combination of communication types) between network addresses.

The computing system can include clients and servers. A client and server are generally remote from each other and typically interact through a communication network. The relationship of client and server arises by virtue of computer programs running on the respective computers and having a client-server relationship to each other.

While this specification contains many specific implementation details, these should not be construed as limitations on the scope of any invention or on the scope of what may be claimed, but rather as descriptions of features that may be specific to particular implementations of particular inventions. Certain features that are described in this specification in the context of separate implementations can also be implemented, in combination, in a single implementation. Conversely, various features that are described in the context of a single implementation can also be implemented in multiple implementations, separately, or in any suitable sub-combination.

Particular implementations of the subject matter have been described. Other implementations, alterations, and permutations of the described implementations are within the scope of the following claims as will be apparent to those skilled in the art. While operations are depicted in the drawings or claims in a particular order, this should not be understood as requiring that such operations be performed in the particular order shown or in sequential order, or that all illustrated operations be performed (some operations may be considered optional), to achieve desirable results. In certain circumstances, multitasking or parallel processing (or a combination of multitasking and parallel processing) may be advantageous and performed as deemed appropriate.

Moreover, the separation or integration of various system modules and components in the described implementations should not be understood as requiring such separation or integration in all implementations, and it should be understood that the described program components and systems can generally be integrated together in a single software product or packaged into multiple software products.

Accordingly, the example implementations do not define or constrain this disclosure.

Furthermore, any claimed implementation is considered to be applicable to at least a computer-implemented method; a non-transitory, computer-readable medium storing computer-readable instructions to perform the computer-implemented method; and a computer system comprising a computer memory interoperably coupled with a hardware processor configured to perform the computer-implemented method or the instructions stored on the non-transitory, computer-readable medium.

## Claims

1. A computer-implemented method (100), comprising:
receiving (102) an image (200a) associated with a core sample of rock (200b);
detecting (112), by a computer, a plurality of areas in the received image, the plurality of areas corresponding to areas at a plurality of depths;
determining (114) a plurality of values of a set of image attributes for the plurality of areas in the received image (200a), wherein the set of image attributes is selected based on correlations between the image attributes and core sample properties to be estimated;
recording the determined plurality of values in one or more pseudo logs of image attributes (400), wherein the one or more pseudo logs include the plurality of values of the set of image attributes at different depths;
determining (106) a core description of the core sample from a set of core descriptions, wherein
i) the core description describes the core sample properties (200b) to be estimated,
ii) the set of core descriptions are associated with a set of training core samples,
iii) each training core sample has a corresponding core description and is associated with one or more pseudo logs of image attributes, and
iv) determining (106) the core description of the core sample (200b) is based on a comparison between the one or more pseudo logs of image attributes associated with the core sample and the one or more pseudo logs of image attributes associated with the set of training core samples; and
providing (108) the core description of the core sample (200b) to an output device.

2. The method (100) of claim 1, wherein each of the plurality of areas represents an area in which a property of the one or more properties of the core sample is substantially homogeneous, and the plurality of areas can be overlapping.

3. The method (100) of claim 1, wherein the comparison between the plurality of values associated with the core sample and sets of plurality of values associated with the set of training core samples includes determining a difference between the plurality of values and at least one set of plurality of values associated with at least one training core sample.

4. The method (100) of claim 1, wherein determining the core description of the core sample includes providing a core description of a training core sample.

5. A non-transitory, computer-readable medium storing one or more instructions executable by a computer-implemented system (500) to perform operations comprising the method of any one of claims 1 to 4.

6. A computer-implemented system (500), comprising:
a computer memory (506); and
a hardware processor (505) interoperably coupled with the computer memory (506) and configured to perform operations comprising the method of any one of claims 1 to 4.

## Patentansprüche

1. Computerimplementiertes Verfahren (100), das Folgendes umfasst:
Empfangen (102) eines Bilds (200a), das mit einem Bohrkern aus Gestein (200b) assoziiert ist;
Detektieren (112), durch einen Computer, mehrerer Bereiche in dem empfangenen Bild, wobei die mehreren Bereiche Bereichen in mehreren Tiefen entsprechen;
Bestimmen (114) mehrerer Werte eines Satzes von Bildattributen für die mehreren Bereiche in dem empfangenen Bild (200a), wobei der Satz von Bildattributen basierend auf Korrelationen zwischen den Bildattributen und zu schätzenden Bohrkerneigenschaften ausgewählt wird;
Aufzeichnen der bestimmten mehreren Werte in einem oder mehreren Pseudologs von Bildattributen (400), wobei das eine oder die mehreren Pseudologs die mehreren Werte des Satzes von Bildattributen bei unterschiedlichen Tiefen beinhalten;
Bestimmen (106) einer Kernbeschreibung des Bohrkerns aus einem Satz von Kernbeschreibungen, wobei
i) die Kernbeschreibung die zu schätzenden Bohrkerneigenschaften (200b) beschreibt,
ii) der Satz von Kernbeschreibungen mit einem Satz von Trainingsbohrkernen assoziiert wird,
iii) jeder Trainingsbohrkern eine entsprechende Kernbeschreibung aufweist und mit einem oder mehreren Pseudologs von Bildattributen assoziiert ist und
iv) Bestimmen (106), dass die Kernbeschreibung des Bohrkerns (200b) auf einem Vergleich zwischen dem einen oder den mehreren Pseudologs von Bildattributen, die mit dem Bohrkern assoziiert sind, und dem einen oder den mehreren Pseudologs von Bildattributen, die mit dem Satz von Trainingsbohrkernen assoziiert sind, basiert; und
Bereitstellen (108) der Kernbeschreibung des Bohrkerns (200b) an eine Ausgabevorrichtung.

2. Verfahren (100) nach Anspruch 1, wobei jeder der mehreren Bereiche einen Bereich darstellt, in dem eine Eigenschaft der einen oder der mehreren Eigenschaften des Bohrkerns im Wesentlichen homogen ist, und sich die mehreren Bereiche überschneiden können.

3. Verfahren (100) nach Anspruch 1, wobei der Vergleich zwischen den mehreren Werten, die mit dem Bohrkern assoziiert sind, und Sätzen mehrerer Werte, die mit dem Satz von Trainingsbohrkernen assoziiert sind, Bestimmen einer Differenz zwischen den mehreren Werten und mindestens einem Satz mehrerer Werte, die mit mindestens einem Trainingsbohrkern assoziiert sind, beinhaltet.

4. Verfahren (100) nach Anspruch 1, wobei das Bestimmen der Kernbeschreibung des Bohrkerns Bereitstellen einer Kernbeschreibung eines Trainingsbohrkerns beinhaltet.

5. Nichtflüchtiges computerlesbares Medium, das eine oder mehrere Anweisungen speichert, die durch ein computerimplementiertes System (500) ausgeführt werden können, um Operationen durchzuführen, die das Verfahren nach einem der Ansprüche 1 bis 4 umfassen.

6. Computerimplementiertes System (500), das Folgendes umfasst:
einen Computerspeicher (506); und
einen Hardwareprozessor (505) der mit dem Computerspeicher (506) betriebsgekoppelt ist und zum Durchführen von Operationen, die das Verfahren nach einem der Ansprüche 1 bis 4 umfassen, ausgelegt ist.

## Revendications

1. Procédé mis en œuvre par ordinateur (100), comprenant :
la réception (102) d'une image (200a) associée à un échantillon carotté de roche (200b) ;
la détection (112), par un ordinateur, d'une pluralité de zones dans l'image reçue, la pluralité de zones correspondant à des zones à une pluralité de profondeurs ;
la détermination (114) d'une pluralité de valeurs d'un ensemble d'attributs d'image pour la pluralité de zones dans l'image reçue (200a), l'ensemble d'attributs d'image étant sélectionné en fonction de corrélations entre les attributs d'image et des propriétés d'échantillon carotté à estimer ;
l'enregistrement de la pluralité déterminée de valeurs dans une ou plusieurs pseudo-diagraphies d'attributs d'image (400), les une ou plusieurs pseudo-diagraphies comportant la pluralité de valeurs de l'ensemble d'attributs d'image à différentes profondeurs ;
la détermination (106) d'une description de carotte de l'échantillon carotté parmi un ensemble de descriptions de carottes, dans lequel
i) la description de carotte décrit les propriétés d'échantillon carotté (200b) à estimer,
ii) l'ensemble de descriptions de carotte est associé à un ensemble d'échantillons carottés d'apprentissage,
iii) chaque échantillon carotté d'apprentissage a une description de carotte correspondante et est associé à une ou plusieurs pseudo-diagraphies d'attributs d'image, et
iv) la détermination (106) de la description de carotte de l'échantillon carotté (200b) est basée sur une comparaison entre les une ou plusieurs pseudo-diagraphies d'attributs d'image associées à l'échantillon carotté et les une ou plusieurs pseudo-diagraphies d'attributs d'image associées à l'ensemble d'échantillons carottés d'apprentissage ; et
la fourniture (108) de la description de carotte de l'échantillon carotté (200b) à un dispositif de sortie.

2. Procédé (100) selon la revendication 1, dans lequel chacune de la pluralité de zones représente une zone dans laquelle une propriété des une ou de plusieurs propriétés de l'échantillon carotté est sensiblement homogène, et les zones de la pluralité de zones peuvent se chevaucher.

3. Procédé (100) selon la revendication 1, dans lequel la comparaison entre la pluralité de valeurs associées à l'échantillon carotté et des ensembles de pluralité de valeurs associés à l'ensemble d'échantillons carottés d'apprentissage comprend la détermination d'une différence entre la pluralité de valeurs et au moins un ensemble de pluralité de valeurs associé à au moins un échantillon carotté d'apprentissage.

4. Procédé (100) selon la revendication 1, dans lequel la détermination de la description de carotte de l'échantillon carotté comprend la fourniture d'une description de carotte d'un échantillon carotté d'apprentissage.

5. Support non transitoire, lisible par ordinateur, stockant une ou plusieurs instructions exécutables par un système mis en œuvre par ordinateur (500) pour réaliser des opérations comprenant le procédé selon l'une quelconque des revendications 1 à 4.

6. Système mis en œuvre par ordinateur (500), comprenant :
une mémoire d'ordinateur (506) ; et
un processeur matériel (505) couplé de manière interopérable avec la mémoire d'ordinateur (506) et configuré pour réaliser des opérations comprenant le procédé selon l'une quelconque des revendications 1 à 4.
